# EUROPEAN PATENT SPECIFICATION

(11) **EP 2 606 369 B1**
(45) Date of publication and mention of the grant of the patent: **11.11.2020**
(21) Application number: 11751815.9
(22) Date of filing: 16.08.2011
(51) Int. Cl.: G01R 33/563, A61B 5/055, A61B 5/103, A61B 5/085

(54) **APPARATUS AND METHOD FOR GENERATING MECHANICAL WAVES INTO LIVING BODIES, SYSTEM AND METHOD FOR MAPPING AN ORGAN OR TISSUE AND SYSTEM AND METHOD FOR CHARACTERISING THE MECHANICAL PROPERTIES OF SAID ORGAN OR TISSUE**
VORRICHTUNG UND VERFAHREN ZUR ERZEUGUNG MECHANISCHER WELLEN IN LEBENDEN KÖRPERN, SYSTEM UND VERFAHREN ZUR ABBILDUNG EINES ORGANS ODER EINES GEWEBES SOWIE SYSTEM UND VERFAHREN ZUR CHARAKTERISIERUNG DER MECHANISCHEN EIGENSCHAFTEN DES BESAGTEN ORGANS ODER GEWEBES
APPAREIL ET PROCÉDÉ POUR GÉNÉRER DES ONDES MÉCANIQUES DANS DES CORPS VIVANTS, SYSTÈME ET PROCÉDÉ POUR CARACTÉRISER LES PROPRIÉTÉS MÉCANIQUES DUDIT ORGANE OU TISSU

(30) Priority: 17.08.2010 EP 10290448
(43) Date of publication of application: 26.06.2013
(73) Proprietor: Université Paris-Saclay, 91190 Saint-Aubin (FR)
(72) Inventor: MAITRE, Xavier, François, F-94120 Fontenay-sous-Bois (FR); DARRASSE, Luc, F-75015 Paris (FR); SINKUS, Ralph, F-95620 Parmain (FR); LOUIS, Bruno, Charles, F-94000 Creteil (FR)
(74) Representative: Grosset-Fournier, Chantal Catherine
(86) International application number: PCT/EP2011/004094
(87) International publication number: WO 2012/022458

(56) References cited:
- DE-A1-102006 062 763
- US-A1- 2002 095 087
- US-A1- 2006 264 736
- US-A1- 2009 299 168
- US-A1- 2010 045 289
- O'BRIEN W D JR ET AL: "Ultrasonic assessment of tissue anisotropy", ULTRASONICS SYMPOSIUM, 1995. PROCEEDINGS., 1995 IEEE SEATTLE, WA, USA 7-10 NOV. 1995, NEW YORK, NY, USA,IEEE, US, vol. 2, 7 November 1995 (1995-11-07), pages 1145-1148, XP010157489, DOI: 10.1109/ULTSYM.1995.495763 ISBN: 978-0-7803-2940-9

## Description

The present invention relates to an apparatus and method for generating mechanical waves into human or animal organs or tissues. It also relates to a system and method for mapping an organ or tissue and system and method for characterising the mechanical properties of said organ or tissue.

The present invention relates to the field of magnetic resonance imaging (MRI) and, more specifically, to devices and methods for implementing magnetic resonance elastography (MRE).

Over the last fourteen years, MRE has become a useful non invasive technique to determine the mechanical properties of human or animal organs or tissues. MRE provides additional valuable diagnostic means to differentiate healthy and diseased tissues. It was successfully applied to characterize tumors in the breast and fibrosis in the liver. This emerging technique effectively extends palpation to remote -organs or tissues that physicians cannot directly access provided mechanical waves can be generated in said organs or tissues.

By measuring the induced oscillating tissue displacements over time, MRE characterizes the induced mechanical wave which propagates in the targeted organs or tissues and which locally depends on the mechanical properties of said organs or tissues. The sensitivity of the technique relies both on the hardware and software capabilities of the MRI unit and on the local wave amplitude as produced in the said organs or tissues.

In many applications, mechanical waves are produced by physically vibrating the surface of the subject or animal with electromechanical or piezoelectric devices. A number of different vibrators have been developed to produce the mechanical waves required to perform MRE. For the breast and liver, mechanical waves may be produced by directly applying a vibrator onto the skin. For the brain, the head may be periodically tilted in a head-rocker system or the subject may bite a vibrating bar to yield propagating waves in the brain.

The known systems offer limited comfort for the subject since they imply vibrating the body or physically hitting the body. Besides, the propagation of the mechanical waves through the body tissues and bones is difficult such that the mechanical wave is largely attenuated before it reaches the targeted organ or tissue. Hence, the targeted organ or tissue are not efficiently vibrated and MRE outcomes are reduced. US 2009/0299168 A1, US 2010/0045289 A1, DE 10 2006 062 763 A1, US 2006/0264736 A1, and O'BRIEN W D JR ET AL: "Ultrasonic assessment of tissue anisotropy",ULTRASONICS SYMPOSIUM, 1995. PROCEEDINGS., 1995 IEEE SEATTLE, WA, USA 7-10 NOV. 1995, NEW YORK, NY, USA,IEEE, US, vol. 2, 7 November 1995 (1995-11-07), pages 1145-1148, XP010157489,DOI: 10.1109/ULTSYM.1995.495763 disclose devices and methods according to the preambles of the independent claims 1 and 9, respectively.

It is an object of the present invention to provide a method and an apparatus in order to efficiently vibrate a human or animal organ or tissue by generating mechanical waves with larger amplitudes therein.

It is an object of the present invention to provide a method and an apparatus to vibrate a human or animal organ or tissue that is easier to implement in the MRE environment and more comfortable for the subject.

It is an object of the present invention to provide a system and method for mapping a human or animal organ or tissue without any undesired artifact from the vibration method and apparatus.

It is another object of the present invention to provide a system and method for characterizing the mechanical properties of said organ or tissue with increased sensitivity.

The invention is disclosed as recited in the appended claims.

Such objects are accomplished through an apparatus for inducing a mechanical wave in at least one region and/or organ and/or tissue of a human or animal body as recited in claim 1.

According to the invention, the generated wave is transmitted to the human or animal body in a gaseous medium without a mechanical transmission by means of solid media.

The present invention makes it possible to excite an organ and/or a tissue and/or a region of a human or animal subject with a mechanical wave in a more comfortable fashion for the subject. Indeed the apparatus according to the invention makes it possible to transmit a mechanical wave to an organ or tissue of a subject without any physical hit or friction on the subject's body or without making the whole body or skull of the subject vibrate through the MRI table, with a bite-bar, or a head-rocker.

Moreover, the apparatus according to the invention uses natural paths in the subjects body to guide the pressure wave down to the organ or region of interest.

According to the present invention, the amplitude of the mechanical waves propagating through the subject's organ or tissue have larger amplitudes compared to the techniques of the prior art.

The apparatus according to the present invention is less complicated, easier to set up, and less intrusive compared to the systems of the prior art.

Moreover, the apparatus makes it possible to more precisely transmit the pressure waves to the organ or tissue.

The apparatus according to the invention may also comprise adapting means, arranged at the extremity of the guiding means at the human or animal body's side, for adapting said extremity of the guiding means to a surface or an airway input of said body.

Hence, the most part of the generated pressure wave may be transmitted from the generating means to the subject's body so the attenuation of the mechanical wave remains limited.

The adapting means may have a shape adapted to any part of the body of said human or animal subject and more specifically to:
- an eye of said human or animal subject,
- the nose of said human or animal subject,
- the mouth of said human or animal subject, or
- the anus of said human or animal subject.

Thus, the pressure wave may be sent to the organ or tissue of the subject *via* the eye, the nose, the mouth, or the anus of the subject.

Internal airway cavities reached through the nose, the mouth, or the anus of the human or animal subject are particularly interesting because they may represent a resonant chamber where the pressure wave could be amplified as more wave energy enters the cavity. Thereof, extra-thoracic upper airways also provide for the pressure wave natural waveguides towards remote organs like, for example, the lung, the hearth, the brain, or even the more remote pituitary gland.

The gaseous medium, in which the pressure wave is generated and guided from the generating means to the subject's body, may be air or any other gas mixture that may be used to ventilate the human or animal subject and which may include labeled gas for medical imaging, like helium-3 or sulflur hexafluoride for MRI.

The means for generating the pressure wave may comprise for example:
- a loudspeaker,
- an electromechanical vibrator, or
- a piezoelectric element.

The generating means may also comprise an amplifier associated with a function generator connected to the loudspeaker, the electromechanical vibrator, or the piezoelectric element.

The waveguide means may comprise a rigid or flexible tubular waveguide, which length and diameter are determined according to the frequency of the pressure wave such that the attenuation of the pressure wave remains very low between the generating means and the subjects' body.

The amplitude of the pressure wave is ultimately set at the generating means such that losses between the generating means and the subjects' body can be compensated.

Advantageously, the apparatus according to the invention may comprise a pressure wave adapter adapting the output of the generating means to the input of the waveguide means.

Such an adapter is needed when there is a difference in the dimensions or the shape of the generator, for example a loudspeaker, and the waveguide to limit impedance mismatch and power losses on the way to the subjects' body.

The invention also provides a system for mapping of at least one region and/or tissue and/or organ of the body of a human or animal subject, said system comprising:
- an apparatus according to the invention for vibrating said organ and/or tissue and/or region,
- magnetic resonance imaging means for imaging the displacements of said organ and/or tissue and/or region while said organ and/or tissue and/or region is vibrated.

Magnetic resonance imaging (MRI) means are well known by the person having ordinary skills in the art. Such imaging means will not be detailed here.

According to the invention, when the organ and/or tissue and/or the region is vibrated, MRI means are used to synchronously image the oscillatory displacements of the tissues at different instants of the period of the mechanical wave.

MRI means may take two or three dimensional images of the organ, the tissue, or the region. Thus, the invention provides two or three dimensional synchronised mapping of the displacements of the targeted organ, tissue, region at different instants of period of the mechanical wave.

The spatial resolution of the MRI mapping may be isotropic since there is no *a priori* preferred spatial direction. The spatial resolution is taken according to the mechanical wavelength, which is expected at a given frequency of the mechanical wave in the imaged region, tissue, or organ. For example in the brain, at 50 Hz, the spatial resolution may be chosen between 1×1×1 and 3×3×3 mm³.

The temporal resolution over the period of the mechanical wave may usually be between 1/4 to 1/8 of this period such that four to eight sets of three dimensional displacement maps are acquired. Each set represents a snapshot of the propagation of the mechanical wave through the organ, tissue, or targeted region at different instants over the period of the mechanical wave.

The invention also provides a system for characterising the mechanical properties of at least one region and/or tissue and/or organ of the body of a human or animal subject, said system comprising:
- a system according to the invention providing a set of displacement maps over a given mechanical period of said organ and/or tissue and/or region
- at least one computer executable program for characterising the mechanical properties of said organ and/or tissue and/or region.
The invention also provides a method for inducing a mechanical wave in at least one region and/or tissue and/or organ of a human or animal body as recited in claim 9.

Such a method may be used to excite a human or animal subject's eye, brain, heart, airways, lung, prostate, or uterus, by transmitting the pressure wave to said brain, heart, airways, or lung *via* the mouth or the nose of the subject, to said prostate or uterus *via* the anus of the subject.

The invention also provides a method for mapping an organ and/or tissue and/or region of a human or animal subject's body, said method comprising the following steps:
- exciting said organ and/or tissue and/or region according to the invention,
- magnetic resonance imaging of said organ and/or tissue and/or region while said organ and/or tissue and/or region is excited.

Such a method may be used to map a human or animal subject's eye, brain, heart, airways, lung, prostate, or uterus.

The invention also provides a method for characterizing the mechanical properties of at least one region and/or tissue and/or organ of a human or animal subject's body, said method comprising the following steps:
- mapping tissue displacements of said organ and/or tissue and/or region according to the invention, and
- analysing said displacement maps to characterize the mechanical properties of at least a part of said organ and/or tissue and/or region.

Such a method may be used to characterize the mechanical properties of a human or animal subject's eye, brain, heart, airways, lung, prostate, or uterus.

The invention also provides a method for characterizing an organ and/or tissue and/or region of a human or animal subject's body, said method comprising the following steps:
- mapping tissue displacement fields of said organ and/or region according to the invention
- analysing said displacement fields to characterize the tissue anisotropy or fibre orientation of at least a part of said organ and/or tissue and/or region.

The characterizing method according to the invention may also comprise a step for analysing the displacement fields and tissue anisotropy to characterize the anisotropic mechanical properties of at least a part of said organ and/or tissue and/or region.

The new and inventive features believed characteristics of the invention are set forth in the appended claims. The invention itself, however, as well as a preferred mode of use, further objects and advantages thereof, will best be understood by reference to the following detailed description of an illustrative detailed embodiment when read in conjunction with the accompanying drawings, wherein:
- figure 1 schematically illustrates an apparatus according to the invention;
- figure 2 schematically illustrates a mapping system according to the present invention;
- figure 3 illustrates a system according to the invention for characterizing the mechanical properties of an organ and/or tissue and/or a region of a human or animal subject's body;
- figure 4 schematically illustrates a method for characterizing at least one region of the body and/or organ and/or tissue of a human or animal subject according to the invention method;
- figures 5-7 illustrate the results obtained thanks to the present invention on the brain of a human subject;
- figures 8-10 illustrates the results obtained thanks to the present invention on the pituitary gland of a human subject;
- figure 11 illustrates the results obtained thanks to the present invention on the upper airways of a human subject;
- figures 12-14 illustrate the results obtained thanks to the present invention on preserved Bioquest® pig lungs;
- figures 15-17 illustrate the results obtained *in vivo* on rat brain thanks to the present invention;
- figure 18 schematically illustrates the steps of a method according to the invention;
- figure 19 illustrates, in the acquired central slice of a rat brain, the dependence of the total wave amplitude and the wavelength with respect to the excitation frequency;
- figures 20-22 illustrate the results obtained in the brain of six rats excited at 521 Hz thanks to the invention;
- figures 23 to 26 illustrate the results obtained thanks to the present invention on the brain of a human subject at 43 Hz and 113 Hz;
- figures 27 and 28 illustrate the results obtained with mouth-throat MRE acquisition in humans with guided pressure wave according to the invention; and
- figures 29 and 30 illustrate the results obtained with hyperpolarized helium-3 MRE in rat lungs according to the invention.

In the following specifications, elements common to several figures are referenced through a common identifier.

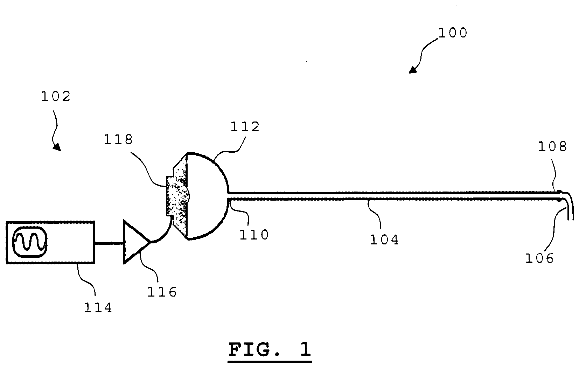

Figure 1 schematically illustrates an example of an apparatus 100 according to the invention.

The apparatus 100 comprises means 102 for generating a pressure wave and a waveguide 104 to guide the pressure wave from the generating means to the subject's body.

The apparatus 100 also comprises an adaptation hose 106 arranged on the extremity 108 of the waveguide 104 at the human or animal body's side, for adapting this extremity 108 of the waveguide 104 to a surface or a cavity of said body, for example to an eye, to the mouth, or to the nose of the subject.

On the other extremity 110 of the waveguide 104, the apparatus comprises a compression hemisphere 112 to match the output of the generating means 102 to the extremity 110 of the waveguide 104.

The means for generating the pressure wave comprise:
- a function generator 114 for generating a burst of electrical sine wave or multiple frequency wave over the frequency range 10-500 Hz;
- an amplifier, more particularly and audio amplifier 116, amplifying the electrical signal generated by the function generator 114, and
- a loudspeaker 118 to produce a pressure wave by transduction of the amplified electrical signal received from the amplifier 116.

The generated pressure wave is then directed to the waveguide 104 by the compression hemisphere 112. The compression hemisphere 112 is connected on the one hand to the output of the loudspeaker 118 and on the other hand to the extremity 110 of the waveguide.

According to a non limitative example, the different elements of the apparatus have the following specifications.
- Function generator 114: Function generator Tektronix AFG 3021B
   ▪ Sine wave in burst mode: 2-10 mVpp at 10-500 Hz
   ▪ Frequency range: 1 µHz-12.5 MHz
   ▪ Harmonic distortion: < -70 dBc for 10 Hz to 20 kHz
   ▪ Function: Generation of MRI-triggered burst of sine wave or multiple frequency wave over the exploration frequency range - 10-500 Hz here.
   ▪ Constraints: Programmable to allow arbitrary wave shape like multiple frequency wave.
- Audio amplifier 116 : Audio amplifier McCRYPT PA 12000
   ▪ Frequency range: 10 - 30 000 Hz
   ▪ Power RMS at 4 Ω: 2 x 450 W
   ▪ Function: Amplification of the generated wave to supply the loudspeaker with required power.
   ▪ Constraints: Adaptation to the loudspeaker impedance
- Loudspeaker 118: Audio loudspeaker Monacor® SPH-135/AD
   ▪ Diameter: 135 mm
   ▪ Power RMS: 40 W
   ▪ Impedance: 8 A
   ▪ Sensibility: 89 dB/1 W/1 m
   ▪ Efficiency: 0.4 %
   ▪ Frequency range: 39-6000 Hz
   ▪ Resonance frequency: 39 Hz
   ▪ DC Resistance: 5.5 A
   ▪ Equivalent Air Volume: 26 L
   ▪ Maximal linear displacement: 1.75 mm
   ▪ Weight: 1.1 kg
   ▪ Function: Transduction of electrical signal to pressure wave.
   ▪ Requirements: Efficient transduction and large maximal linear displacement over the exploration frequency range to induce accordingly large amplitude pressure wave.
- Compression hemisphere 112: Altuglas® hemisphere
   ▪ Diameter: 200 mm
   ▪ Circular output: 20 mm
   ▪ Function: Adaptation of the loudspeaker-generated pressure wave to the transmission tube cross-section.
   ▪ Requirements: A small volume to maximize compression and a smooth transition shape to limit wave reflexion.
   ▪ Other shapes may be used such as conic, exponential or hyperbolic shapes.
- Waveguide 104 (Transmission tube) Altuglas® tube
   ▪ Length: 1740 mm
   ▪ Inner diameter: 17 mm
   ▪ Outer diameter: 20 mm
   ▪ Function: Transmission of the pressure wave from the magnetic loudspeaker to the imaging site in the magnetic field of the MRI imager.
   ▪ Requirements: The length of the tube must be adapted to the excitation frequency. Its length, in addition to the length of the adaptation hose, must correspond to an odd multiple of a quarter wavelength of the pressure wave such that the amplitude of the pressure wave is maximal at the output.
- Adaptation hose 106: Flexible silicone hose Masterflex®
   ▪ Length: 200 mm
   ▪ Inner diameter: 20 mm
   ▪ Outer diameter: 28 mm
   ▪ Function: Transmission of the pressure wave along different orientations and coupling to the measured system - subject or sample.
   ▪ Requirements: To limit the reflections of the pressure wave, the hose must be adapted to the waveguide (transmission tube) diameter. This diameter must be kept along the different orientations and, for a human subject, under the pressure of the lips and the teeth at the mouth entrance.

For the subject's protection and comfort, a breathing filter (not represented) like an Intersurgical® clear-guard 1644131 and a mouthpiece (not represented) like an Intersurgical® 1930 may be added at the end of the adaptation hose 106 before the subject.

Reducing means (not represented) may be used to adapt the setup to smaller subjects like animals. A non limitative example of such a reducer may have the following specifications: Reducer (not represented): A plastic adaptation piece Intersurgical® 1968
▪ Input diameter: 22 mm
▪ Output diameter: 6 mm
▪ Function: Adaptation of the setup to smaller systems like small animals.
▪ Requirements: A smooth transition shape to limit wave reflexion.

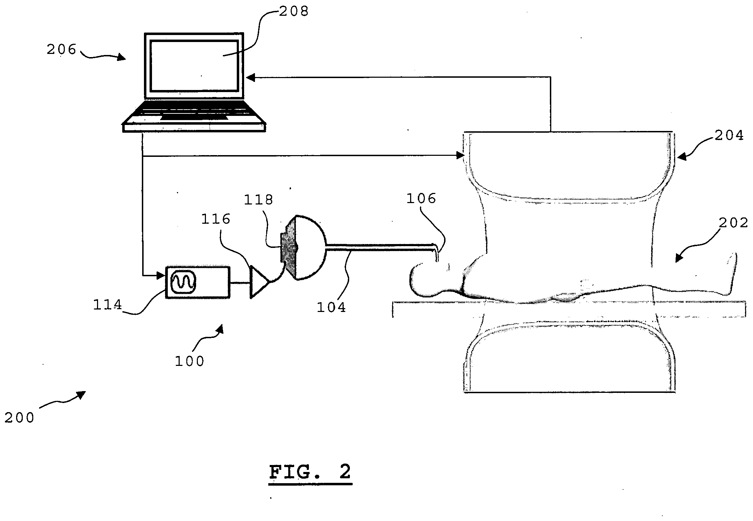

Figure 2 schematically illustrates an example of a mapping system 200 according to the present invention.

The mapping system comprises an apparatus 100 for exciting an organ and/or a region of a subject 202 with a pressure wave as represented on figure 1.

The adaptation hose 106 of the apparatus 100 is put in the mouth of the subject 202.

The subject is placed in magnetic resonance imaging means 204.

Computer means 206 are connected to the function generator 114 and to magnetic resonance imaging means 204.

The computer means 206 control the function generator 114 and the magnetic resonance imaging means 204 so that the function generator 114 and the magnetic resonance imaging means 204 are triggered synchronously.

The pressure wave is generated and is sent to the organ of the subject 202. The pressure wave causes a mechanical wave which propagates in the organ, tissue, or region of the subject. During the propagation of the mechanical wave in the organ, the magnetic resonance imaging means 204 acquire images of said organ, tissue, or region.

The tissue displacements of the targeted organ, tissue, or region of the body is imaged slice by slice. The slices have a thickness of 1.6 to 8 mm, for example 2 mm. A three dimensional displacement map is obtained by combining the images of all slices.

The images are sent to the computer means 206. The computer means comprise a display screen 208 on which the images taken by the magnetic resonance imaging means 204 may be displayed.

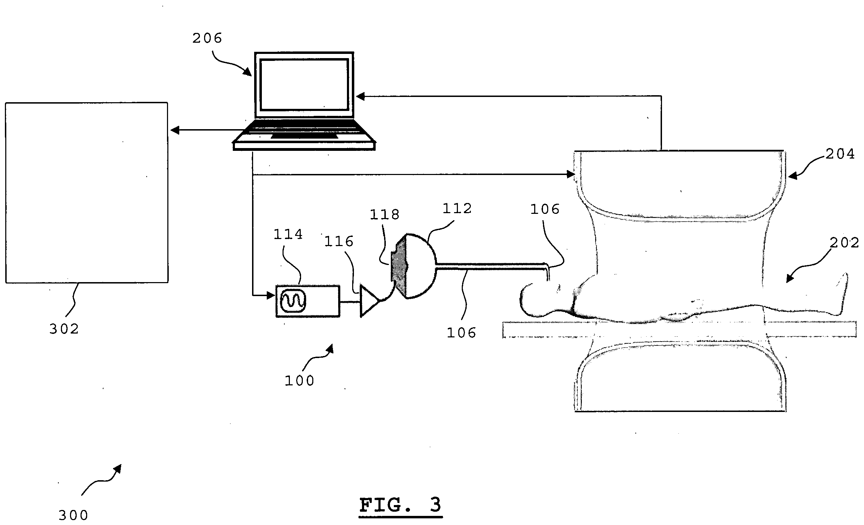

Figure 3 schematically illustrates an example of a system 300 for characterizing the mechanical properties of an organ and/or a tissue and/or a region of a subject's body according to the present invention.

The system 300 comprises a mapping system 200 as represented on figure 2.

The system 300 also comprises a analyzing module 302 for analyzing the images taken by the mapping system 200 and characterizing the mechanical properties of the imaged organ.

The images taken by the magnetic resonance imaging means 202 and sent to the computer means 206 are transferred to the analyzing module 302. In the analyzing module 302, the phase of the images is unwrapped to yield displacement maps at the different instants according to the imaging sequence parameters. Movies of the propagating mechanical waves may then be processed as shown in the presentation of the results. The local wavelength of the mechanical waves is inferred from the displacement maps to finally deduce the viscoelastic moduli of the studied organ, tissue, or region of the subject's body.

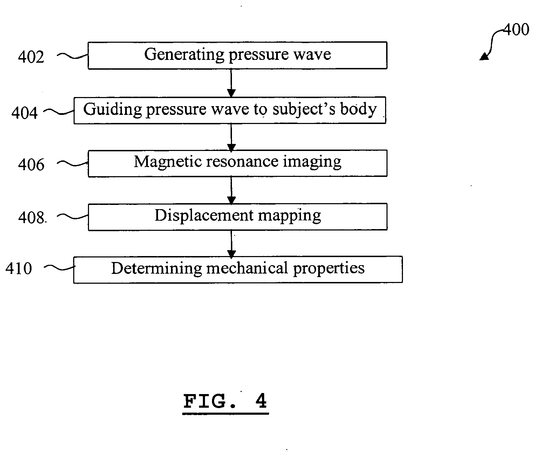

Figure 4 schematically illustrates a method for characterizing at least one region of the body and/or organ and/or tissue of a human or animal subject according to the invention method.

The method 400 of figure 4 comprises a step 402 for generating a pressure wave.

The generated pressure wave is guided from generating means to the body of the subject in a gaseous medium at step 404. This pressure wave generates mechanical displacements in the subjects body in the targeted region, organ and/or tissue.

The targeted region, organ and/or tissue is imaged with magnetic resonance imaging means at step 406.

The taken images are then analyzed at step 408 to realize a displacement mapping of the targeted region, organ and/or tissue.

The displacement mapping in/of/around the targeted region is analysed in step 410 to determine the mechanical properties of the targeted region, organ and/or tissue.

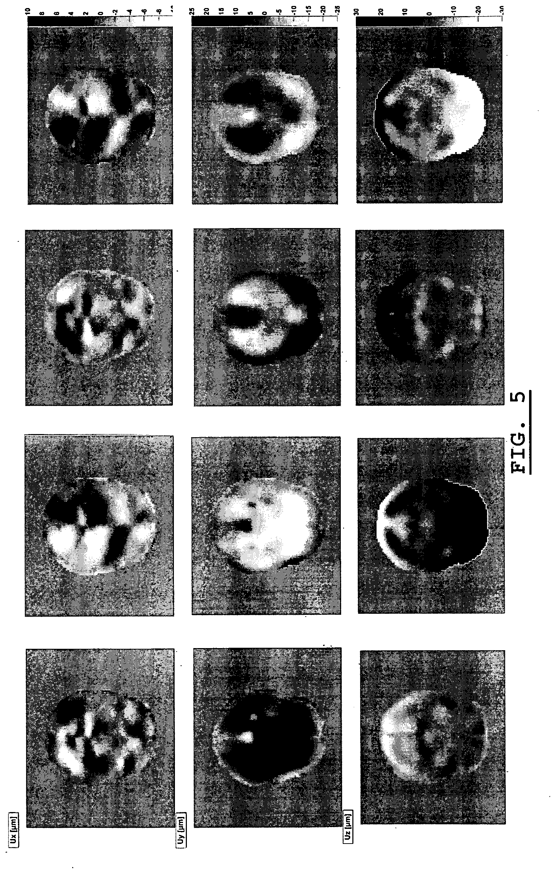

Figures 5-7 illustrate the results obtained thanks to the present invention on the brain of a human subject.

Figure 5 illustrates displacement maps along the three motion encoded directions (Uₓ, U_{y}, and U_{z} in *µ*m) in a central slice of the brain of a healthy subject at four over eight different instants of the mechanical cycle at 50 Hz.

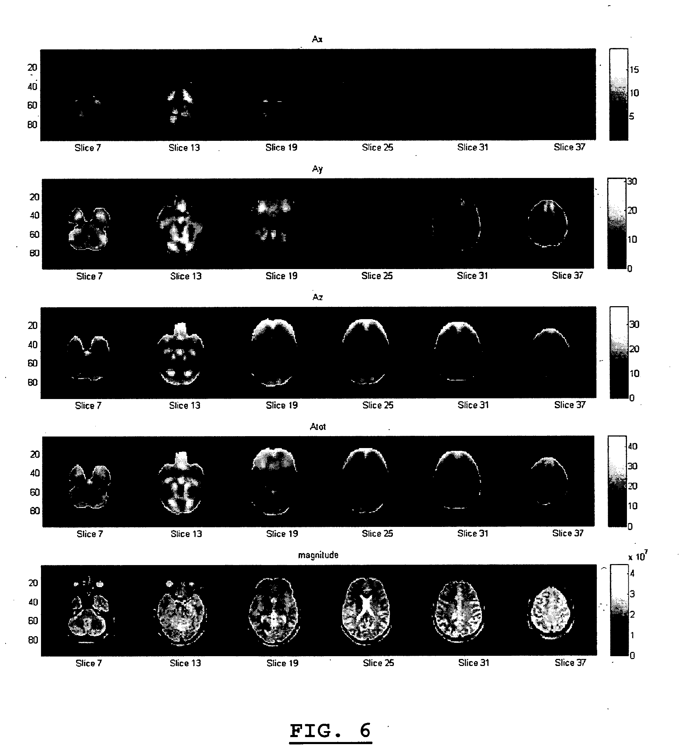

Figure 6 illustrates wave amplitudes given along the three motion encoded directions (AX, AY, AZ) as well as the resulting total amplitude (Atot) in *µ*m for six over 43 acquired slices in a full brain MRE acquisition. The corresponding average magnitude image is also given for reference (bottom row) in arbitrary units. Field of view=146×256×129 mm³, voxel=3×3×3 mm³, TR=4301 ms, 8 dynamics.

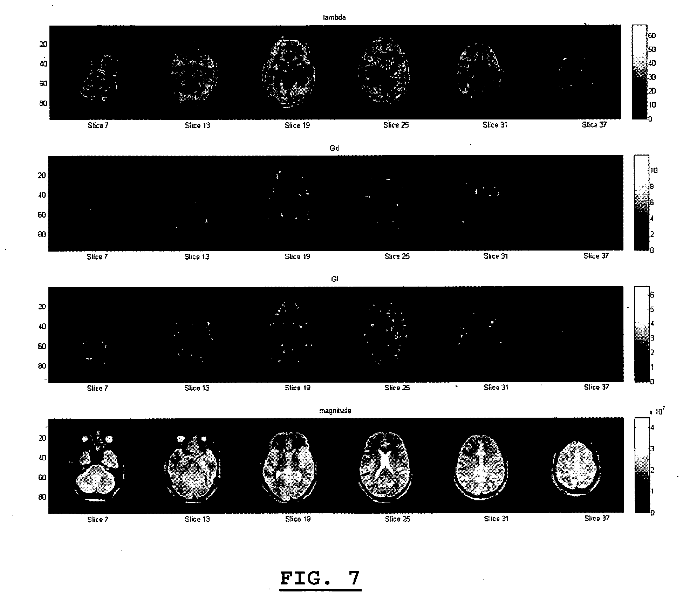

Figure 7 illustrates maps of corresponding processed wavelength (in mm), dynamic shear modulus (G_{d} in kPa), and loss shear modulus (Gₗ in kPa) given with the corresponding average magnitude image as a reference (bottom row).

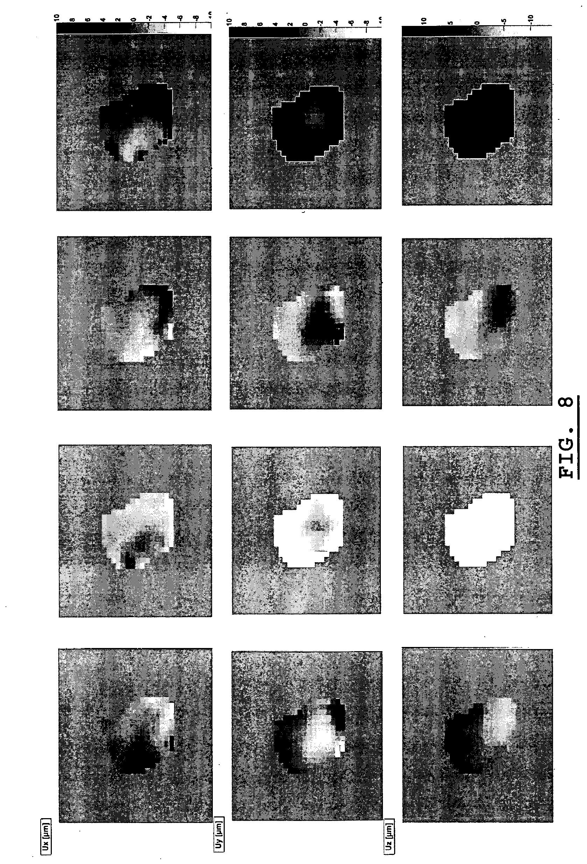

Figures 8-10 illustrates the results obtained thanks to the present invention on the pituitary gland of a human subject.

Figure 8 illustrates displacement maps along the three motion encoded directions (Uₓ, U_{y}, and U_{z} in *µ*m) in a central slice of the pituitary of a healthy subject at four over eight different instants of the mechanical cycle at 126 Hz.

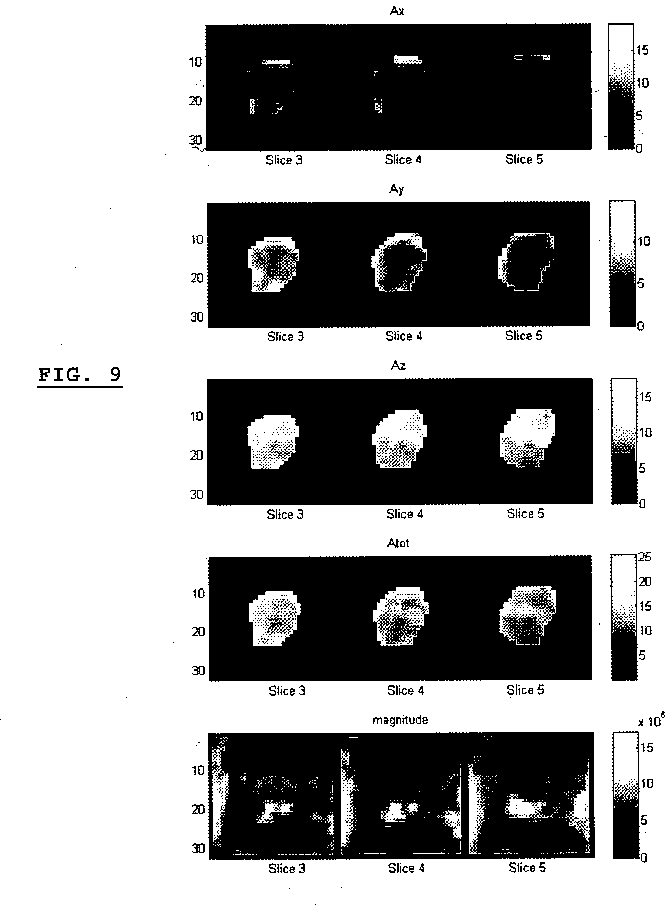

Figure 9 illustrates wave amplitudes given along the three motion encoded directions (AX, AY, AZ) as well as the resulting total amplitude (Atot) in *µ*m for 3 over 7 acquired slices in a pituitary MRE acquisition. The corresponding average magnitude image is also given for reference (bottom row) in arbitrary units. Field of view=32×32×32 mm³, voxel=1.4×1.4×1.4 mm³, TR=889 ms, 8 dynamics.

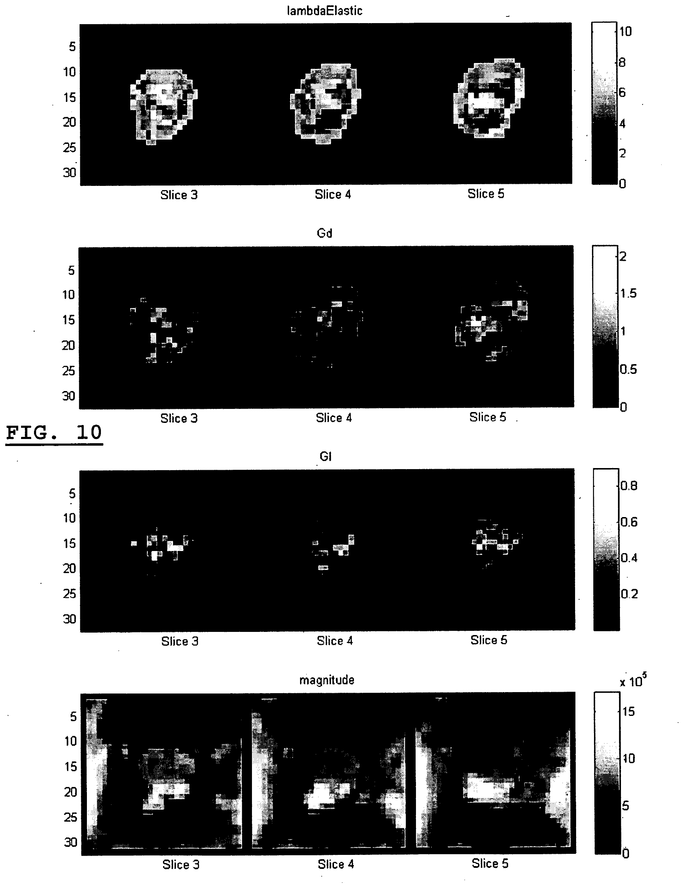

Figure 10 illustrates maps of corresponding processed wavelength (in mm), dynamic shear modulus (G_{d} in kPa), and loss shear modulus (Gₗ in kPa) given with the corresponding average magnitude image as a reference (bottom row).

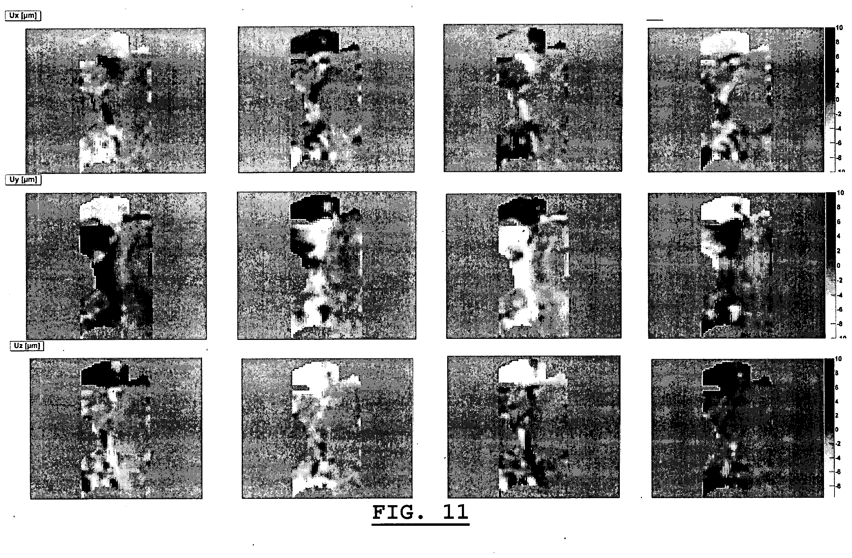

Figure 11 illustrates the results obtained thanks to the present invention on the upper airways of a human subject. Figure 10 illustrates displacement maps along the three motion encoded directions (Uₓ, U_{y}, and U_{z} in *µ*m) in a central slice of the upper airways (from the mouth down to the trachea) of a healthy subject at four over eight different instants of the mechanical cycle at 54 Hz.

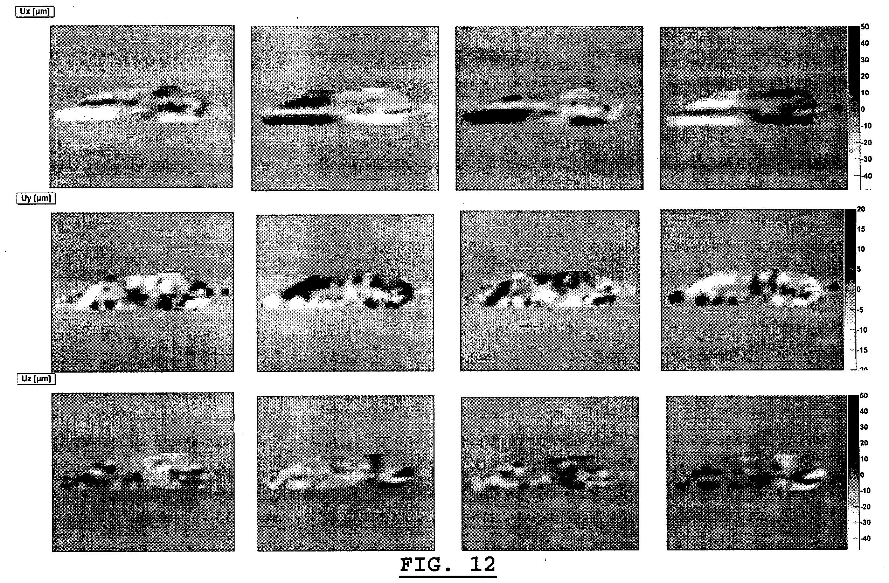

Figures 12-14 illustrate the results obtained thanks to the present invention on preserved Bioquest® pig lungs.

Figure 12 illustrates displacement maps along the three motion encoded directions (Uₓ, U_{y}, and U_{z} in *µ*m) in a central slice of the lung of a healthy subject at four over eight different instants of the mechanical cycle at 140 Hz.

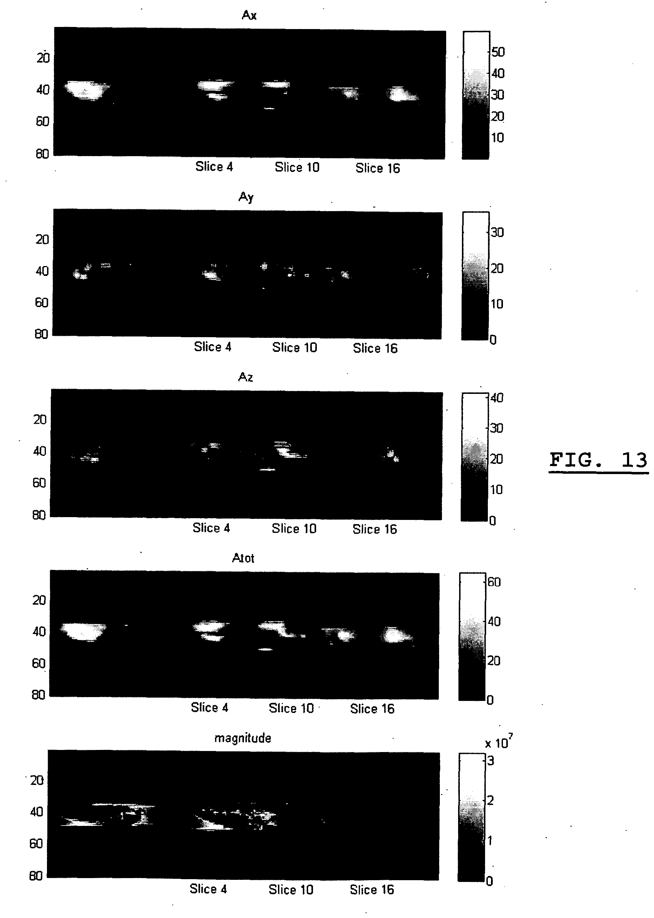

Figure 13 illustrates wave amplitudes, given along the three motion encoded directions (AX, AY, AZ) as well as the resulting total amplitude (Atot) in *µ*m for 3 over 20 acquired slices in a full lung MRE acquisition. The corresponding average magnitude image is also given for reference (bottom row) in arbitrary units. Field of view=320×320×80 mm³, voxel=4×4×4 mm³, TR=857 ms, 8 dynamics.

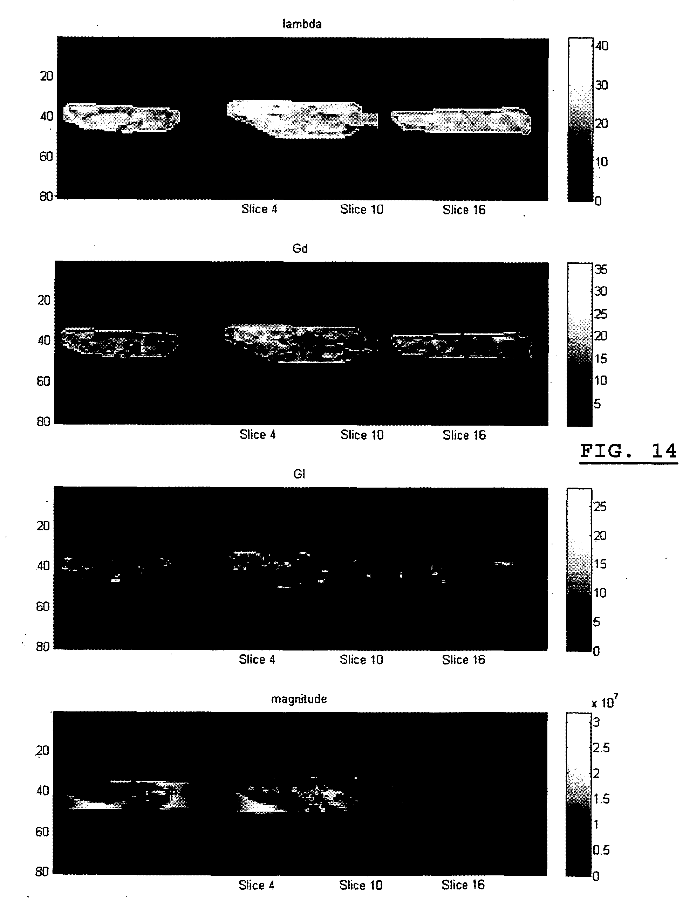

Figure 14 illustrates maps of corresponding processed wavelength (in mm), dynamic shear modulus (G_{d} in kPa), and loss shear modulus (Gₗ in kPa) given with the corresponding average magnitude image as a reference (bottom row).

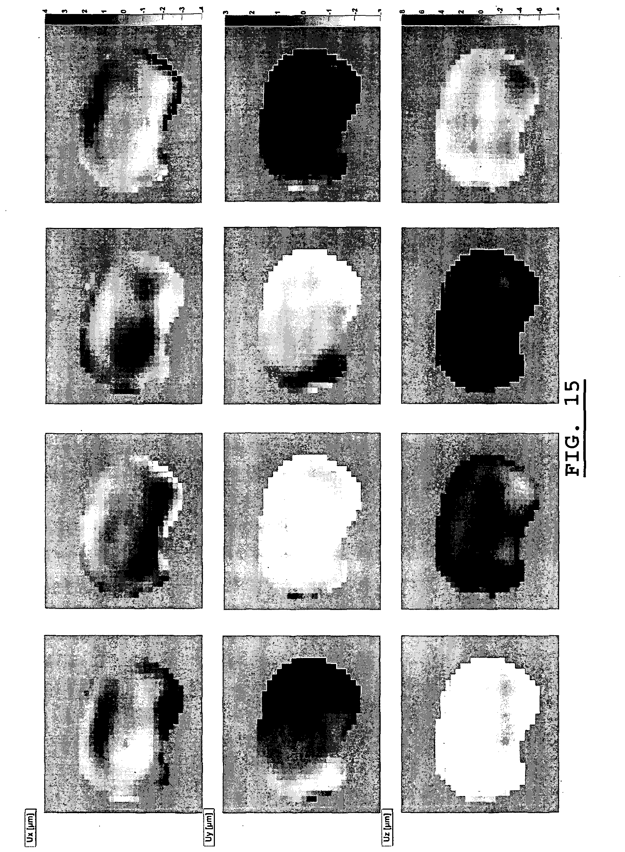

Figures 15-17 illustrate the results obtained *in vivo* on rat brain thanks to the present invention.

Figure 15 illustrates displacement maps along the three motion encoded directions (Uₓ, U_{y}, and U_{z} in *µ*m) in a central slice of the brain of a healthy animal at four over eight different instants of the mechanical cycle at 520 Hz.

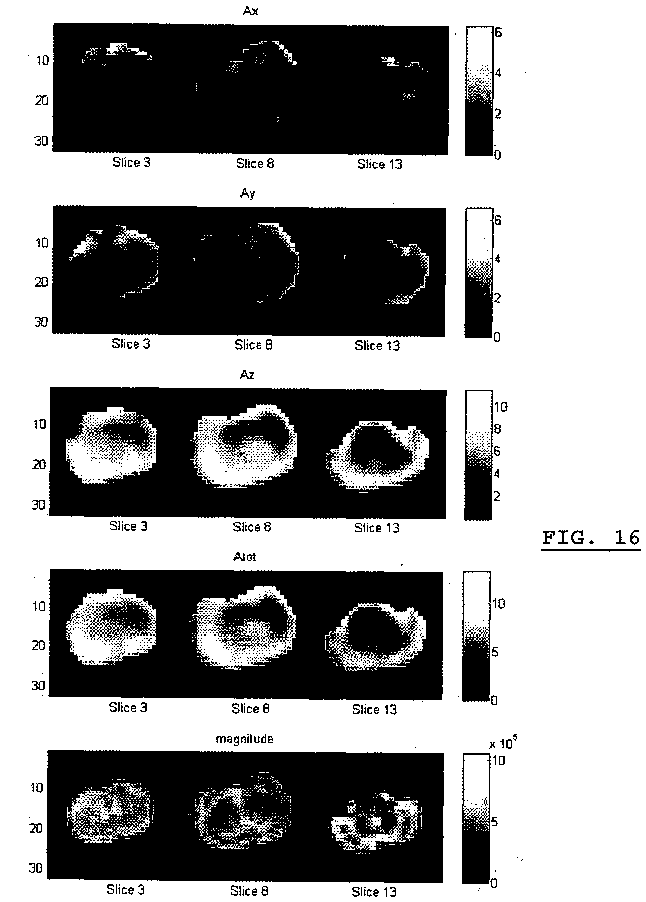

Figure 16 illustrates wave amplitudes given along the three motion encoded directions (AX, AY, AZ) as well as the resulting total amplitude (Atot) in *µ*m for six over 20 acquired slices in a full brain MRE acquisition. The corresponding average magnitude image is also given for reference (bottom row) in arbitrary units. Field of view=20×20×17 mm³, voxel=0.8×0.8×0.8 mm³, TR=2937 ms, 8 dynamics.

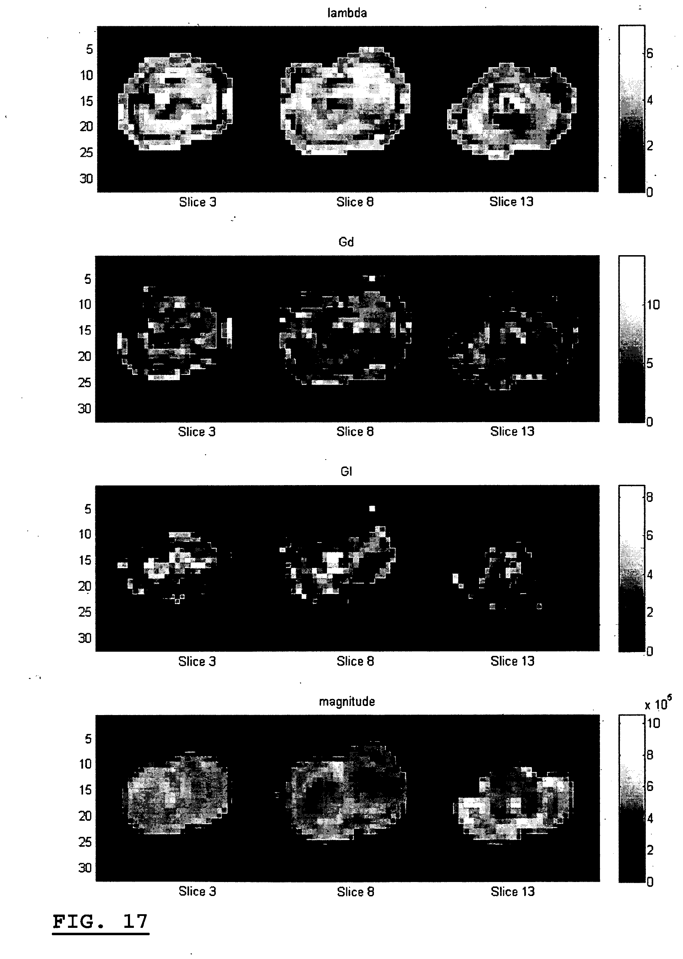

Figure 17 illustrates maps of corresponding processed wavelength (in mm), dynamic shear modulus (G_{d} in kPa), and loss shear modulus (Gₗ in kPa) are given with the corresponding average magnitude image as a reference (bottom row).

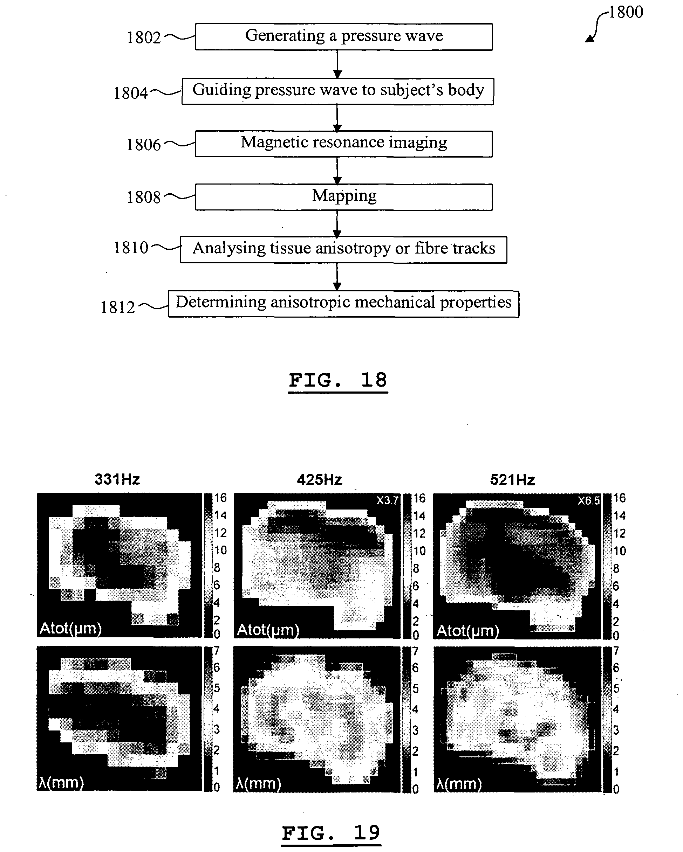

Figure 18 schematically illustrates a method for a characterizing at least one region of the body and/or organ and/or tissue of a human or animal subject according to the invention method.

The method 1800 of figure 18 comprises a step 1802 for generating a pressure wave.

The generated pressure wave is guided from generating means to the body of the subject in a gaseous medium at step 1804. This pressure wave generates mechanical displacements in the subjects body in the targeted region, organ and/or tissue.

The targeted region, organ and/or tissue is imaged with magnetic resonance imaging means at step 1806.

The taken images are then analyzed at step 1808 to realize a displacement mapping of the targeted region, organ and/or tissue.

The displacement mapping is then analysed at step 1810 to determine tissue anisotropy or fibre orientation in/of/around the targeted region, organ and/or tissue.

The displacement mapping and tissue anisotropy in/of/around the targeted region is analysed in step 1810 to determine the anisotropic mechanical properties of the targeted region, organ and/or tissue.

Figure 19 illustrates, in the acquired central slice of a rat brain, the dependence of the total wave amplitude and the wavelength with respect to the excitation frequency at 331 Hz, 425 Hz, and 521 Hz with field of view=20×20×17 mm³, voxel=0.8×0.8×0.8 mm³, TR=2937 ms, 8 dynamics. As expected, the total wave amplitude and the wavelength decrease with the frequency.

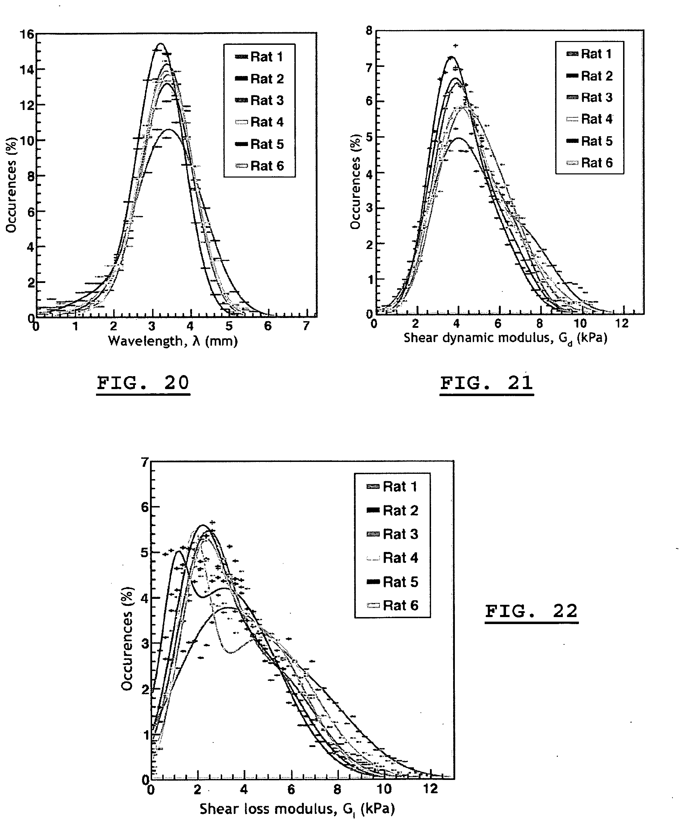

Figures 20-22 illustrate the results obtained in the brain of six rats excited at 521 Hz thanks to the invention. In figures 20-22 bimodal Gaussian fits to the data are added for visualization of the distributions.

More particularly, figure 20 illustrates the reproducibility of the distribution of wavelength obtained in the brain of six rats excited at 521 Hz, figure 21 illustrates the reproducibility of the distribution of shear storage modulus (kPa) obtained in the brain of six rats excited at 521 Hz, and figure 22 illustrates the reproducibility of the distribution of shear loss modulus (kPa) in the brain of six rats excited at 521 Hz.

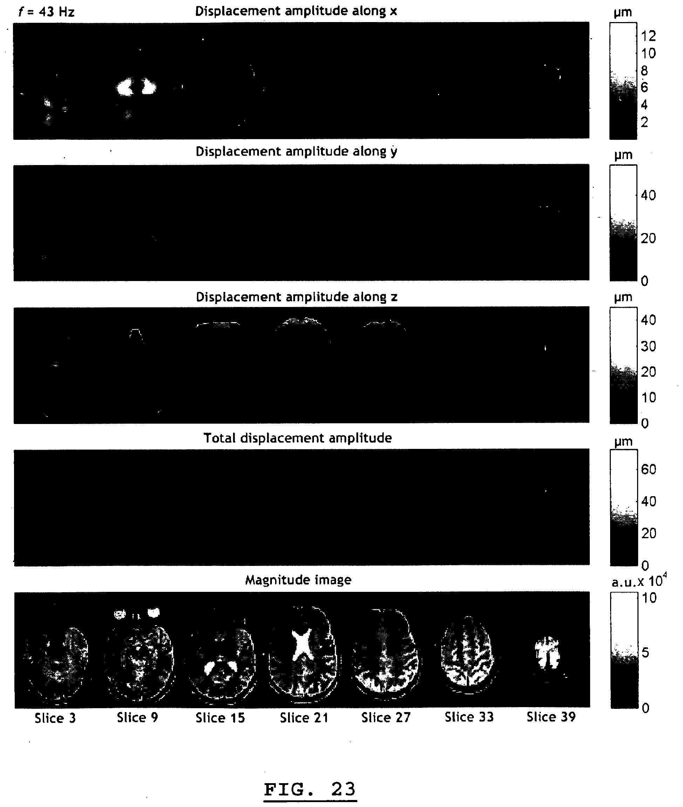

Figures 23 to 26 illustrate the results obtained thanks to the present invention on the brain of a human subject at 43 Hz and 113 Hz.

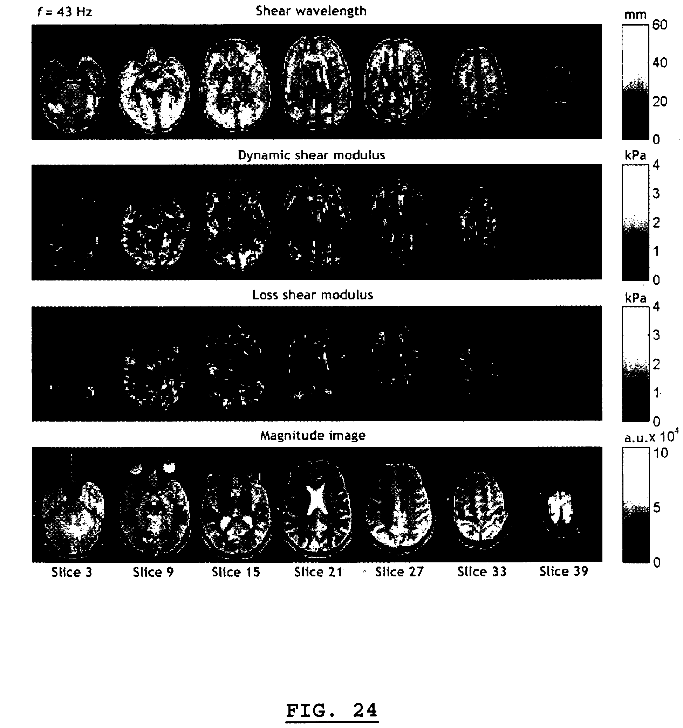

Figure 23 illustrates the displacement amplitudes given along the three motion encoded directions (AX, AY, AZ) as well as the resulting total amplitude (Atot) in *µ*m for seven over 43 acquired slices in a full brain MRE acquisition. The corresponding average magnitude image is also given for reference (bottom row) in arbitrary units with the following parameters: field of view=154×264×118 mm³, voxel=2.75×2.75×2.75 mm³, *f*=43 Hz. Figure 24 illustrates the maps of corresponding processed wavelength (in mm), dynamic shear modulus (G_{d} in kPa), and loss shear modulus (Gₗ in kPa) given with the corresponding average magnitude image as a morphological reference (bottom row) with the following parameters: field of view=154×264×118 mm³, voxel=2.75×2.75×2.75 mm³, *f*=43 Hz.

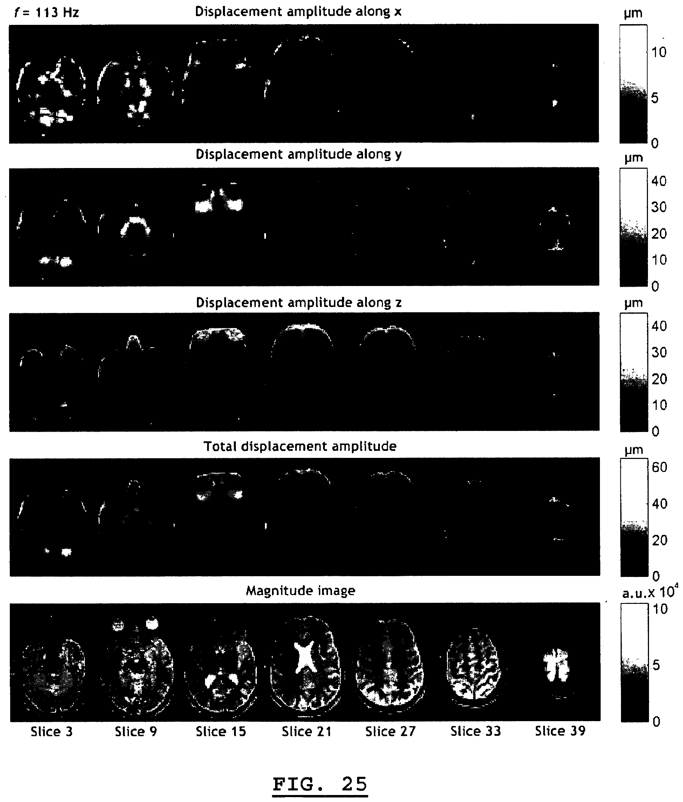

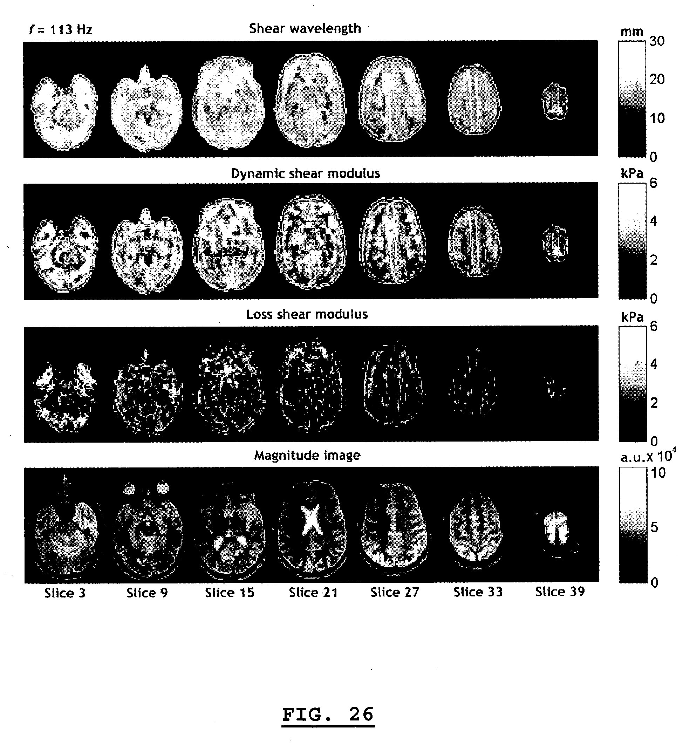

Figure 25 illustrates the displacement amplitudes given along the three motion encoded directions (AX, AY, AZ) as well as the resulting total amplitude (Atot) in *µ*m for seven over 43 acquired slices in a full brain MRE acquisition. The corresponding average magnitude image is also given for reference (bottom row) in arbitrary units, with the following parameters: Field of view=154×264×118 mm³, voxel=2.75×2.75×2.75 mm³, *f*=113 Hz. Figure 26 illustrates the maps of corresponding processed wavelength (in mm), dynamic shear modulus (G_{d} in kPa), and loss shear modulus (Gₗ in kPa) given with the corresponding average magnitude image as a morphological reference (bottom row) with the following parameters: field of view=154×264×118 mm³, voxel=2.75×2.75×2.75 mm³, *f*=113 Hz.

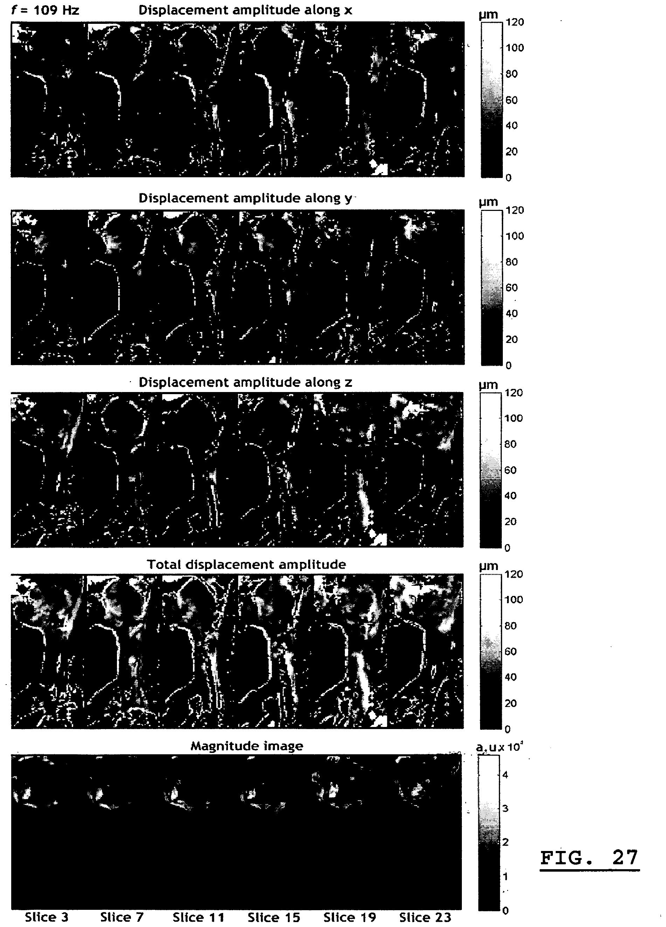

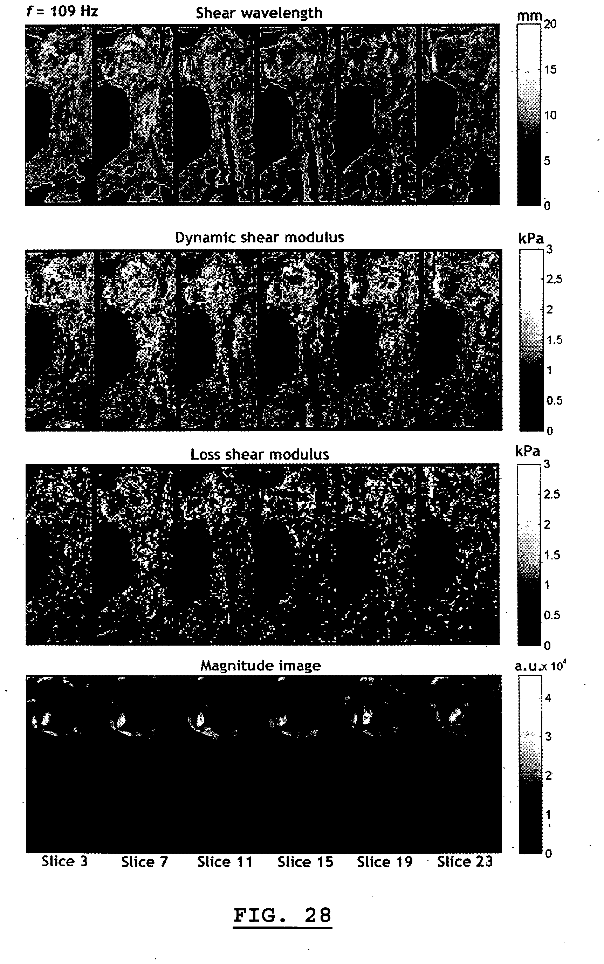

Figures 27 and 28 illustrate the results obtained with mouth-throat MRE acquisition in humans with guided pressure wave according to the invention.

Figure 27 illustrates the displacement amplitudes given along the three motion encoded directions (AX, AY, AZ) as well as the resulting total amplitude (Atot) in *µ*m for six over 28 acquired slices in a full mouth-throat MRE acquisition, with the following parameters: field of view=112×256×56 mm³, voxel=2×2×2 mm³, *f*=109 Hz. The corresponding average magnitude image is also given for reference (bottom row) in arbitrary units. Figure 27 illustrates the maps of corresponding processed wavelength (in mm), dynamic shear modulus (G_{d} in kPa), and loss shear modulus (Gₗ in kPa) given with the corresponding average magnitude image as a morphological reference (bottom row).

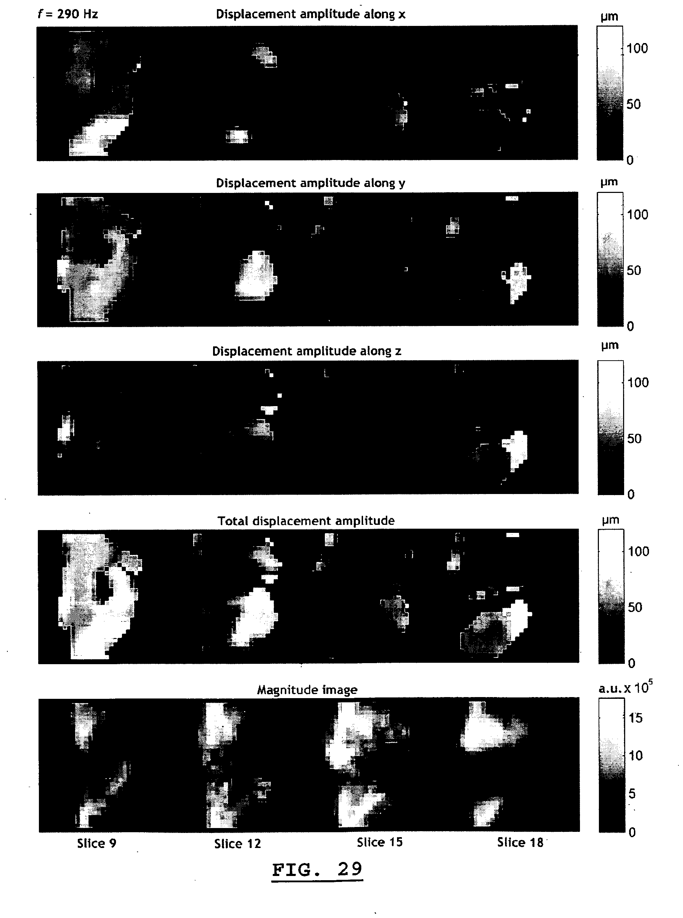

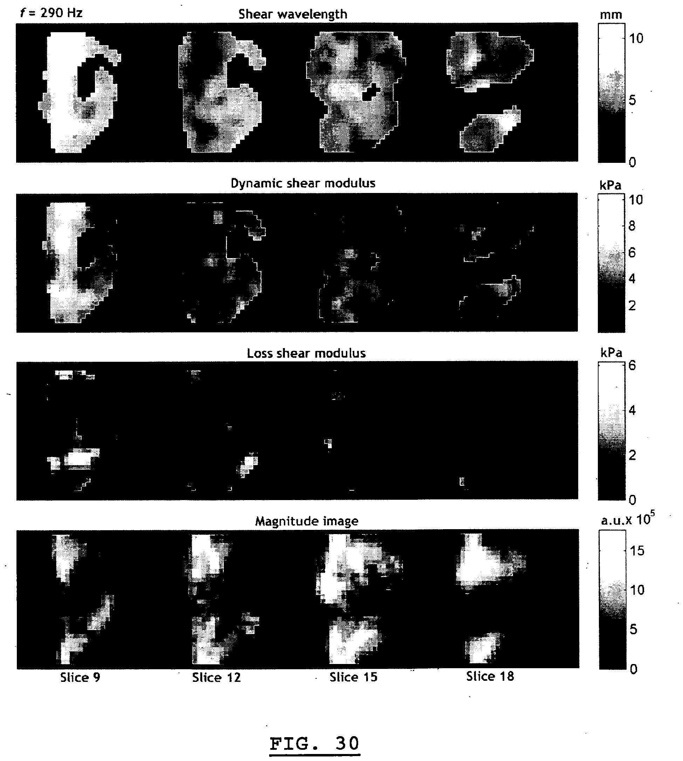

Figures 29 and 30 illustrate the results obtained with hyperpolarized helium-3 MRE in rat lungs according to the invention, with the following parameters: field of view=80×40×30 mm³, voxel=1.25×1.25×1.25 mm³ and *f*=290 Hz.

Figure 29 illustrates the displacement amplitudes given along the three motion encoded directions (AX, AY, AZ) as well as the resulting total amplitude (Atot) in *µ*m for four over 20 acquired slices in a full lung hyperpolarized helium-3 MRE acquisition. The corresponding average magnitude image is also given for reference (bottom row) in arbitrary units. Figure 30 illustrates the maps of corresponding processed wavelength (in mm), dynamic shear modulus (G_{d} in kPa), and loss shear modulus (Gₗ in kPa) given with the corresponding average magnitude image as a morphological reference (bottom row).

The present invention may be applied to the following organs, tissues or parts of a subject's body: eyes, face, brain, neck, airways, lung, heart, prostate, breast, liver, abdomen, etc.

## Claims

1. Apparatus (100) for inducing a mechanical wave in at least one region of the body and/or organ and/or tissue of a human or animal subject (202), said apparatus comprising:
- means (102) for generating a pressure wave of a given frequency in a gaseous medium, and
- waveguide means (104) in the form of a transmission tube for guiding, in air or any other gas mixture that may be used to ventilate the human or animal subject (202), said pressure wave from said generating means (102) to a human or animal body (202),
- an adaptation hose (106) arranged on the extremity (108) of the waveguide (104) at the human or animal body's side, for adapting this extremity (108) of the waveguide (104) to a surface or a cavity of said body, **characterized in that** the generated wave is transmitted to the human or animal body in a gaseous medium without a mechanical transmission by means of solid media.

2. The apparatus (100) according to claim 1, **characterised in that** the gaseous medium includes labeled gas for medical imaging, like helium-3 or sulfur hexafluoride for MRI.

3. The apparatus (100) according to anyone of the preceding claims, **characterised in that** the means for generating a pressure wave comprise:
- a loudspeaker (118),
- an electromechanical vibrator, or
- a piezoelectric element.

4. The apparatus (100) according to anyone of the preceding claims, **characterised in that** the waveguide in the form of a transmission tube is a rigid or flexible tubular waveguide (104), whose length and diameter are determined according to the frequency of the pressure wave.

5. The apparatus (100) according to anyone of the preceding claims, **characterised in that** it also comprises a pressure wave adapter (112) adapting the output of the generating means (102) to the input (110) of the waveguide means (104).

6. The apparatus according to anyone of the claims 1 to 5, **characterised in that** the adaptation hose (106) has a shape adapted to:
- an eye of said human or animal subject,
- the nose of said human or animal subject,
- the mouth of said human or animal subject, or
- the anus of said human or animal subject.

7. System (200) for mapping of at least one region and/or tissue and/or organ of the body of a human or animal subject (202), said system comprising:
- an apparatus (100) according to anyone of the preceding claims for vibrating said organ and/or tissue and/or region,
- magnetic resonance imaging means (204) for imaging the displacements of said organ and/or tissue and/or region while said organ and/or tissue and/or region is vibrated.

8. System (300) for characterizing the mechanical properties of at least one region and/or tissue and/or organ of the body of a human or animal subject (202), said system comprising:
- a system (200) according to claim 7, providing the displacements within said organ and/or tissue and/or region
- at least one computer executable program for analysing said displacements to characterize the mechanical properties of at least a part of said organ and/or tissue and/or region.

9. Method (400) for inducing a mechanical wave in at least one region and/or tissue and/or organ of a human or animal body (202), said method comprising the following steps:
- Generating (402), by generating means (102), a pressure wave of a given frequency in air or any other gas mixture that may be used to ventilate the human or animal subject (202), and
- guiding (404) said pressure wave from said generating means (102) to said human or animal body (202) in air or any other gas mixture that may be used to ventilate the human or animal subject (202) **characterized in that** the generated wave is transmitted to the human or animal body in a gaseous medium without a mechanical transmission by means of solid media.

10. Method (400) for inducing a mechanical wave in at least one region and/or tissue and/or organ of a human or animal body (202) according to claim 9 comprising the step of adapting the length of the tube to the excitation frequency, so that said length of the tube, in addition to the length of the adaptation hose, corresponds to an odd multiple of a quarter wavelength of the pressure wave.

11. Use of a method according to claim 9 to excite a human or animal subject's eye, brain, airways, heart, lung, prostate, or uterus.

12. Method (400) for mapping an organ and/or a tissue and/or a region of a human or animal subject's body (202), said method comprising the following steps:
- vibrating (402, 404) said organ and/or region according to the method according to claim 9,
- magnetic resonance imaging (406, 408) of said organ and/or tissue and/or region while said organ and/or tissue and/or region are vibrated.

13. Use of a method according to claim 12 to map a human or animal subject's eye, brain, airways, heart, lung, prostate, or uterus.

14. Method (400) for characterizing an organ and/or tissue and/or region of a human or animal subject's body (202), said method comprising the following steps:
- mapping (408) tissue displacements of said organ and/or region according to claim 11, and
- analysing (410) said displacement maps to characterize the mechanical properties of at least a part of said organ and/or tissue and/or region.

15. Use of a method according to claim 14 to characterize the mechanical properties of a human or animal subject's eye, brain, airways, heart, lung, prostate, or uterus.

16. Method (1800) for characterizing an organ and/or tissue and/or region of a human or animal subject's body (202), said method comprising the following steps:
- mapping (1802-1808) tissue displacement fields of said organ and/or region according to claim 12, and
- analysing (1810) said displacement fields to characterize the tissue anisotropy or fibre orientation of at least a part of said organ and/or tissue and/or region.

17. Method (1800) according to claim 16, also comprising a step (1812) for analysing the displacement fields and tissue anisotropy to characterize the anisotropic mechanical properties of at least a part of said organ and/or tissue and/or region.

## Patentansprüche

1. Vorrichtung (100) zum Einleiten einer mechanischen Welle in zumindest einen Bereich des Körpers und/oder Organs und/oder Gewebes eines menschlichen oder tierischen Individuums (202), die Vorrichtung umfassend:
- Mittel (102) zum Erzeugen einer Druckwelle mit einer gegebenen Frequenz in einem gasförmigen Medium, und
- Wellenleitermittel (104) in der Form einer Übertragungsröhre zum Leiten, in Luft oder jeglichem anderen Gasgemisch, das zum Beatmen des menschlichen oder tierischen Individuums (202) benutzt werden kann, der Druckwelle vom Erzeugungsmittel (102) zu einem menschlichen oder tierischen Körper (202),
- einen Anpassungsschlauch (106), der am Ende (108) des Wellenleiters (104) auf der Seite des menschlichen oder tierischen Körpers angeordnet ist, zum Anpassen dieses Endes (108) des Wellenleiters (104) an eine Oberfläche oder Höhle des Körpers, **dadurch gekennzeichnet, dass** die erzeugte Welle in einem gasförmigen Medium ohne eine mechanische Übertragung mittels fester Medien zum menschlichen oder tierischen Körper übertragen wird.

2. Vorrichtung (100) nach Anspruch 1, **dadurch gekennzeichnet, dass** das gasförmige Medium markiertes Gas für medizinische Abbildung enthält, wie etwa Helium-3 oder Schwefelhexafluorid für MRT.

3. Vorrichtung (100) nach einem der vorhergehenden Ansprüche, **dadurch gekennzeichnet, dass** die Mittel zum Erzeugen einer Druckwelle umfassen:
- einen Lautsprecher (118),
- einen elektromechanischen Vibrator, oder
- ein piezoelektrisches Element.

4. Vorrichtung (100) nach einem der vorhergehenden Ansprüche, **dadurch gekennzeichnet, dass** der Wellenleiter in der Form einer Übertragungsröhre ein starrer oder flexibler röhrenförmiger Wellenleiter (104) ist, dessen Länge und Durchmesser gemäß der Frequenz der Druckwelle festgelegt ist.

5. Vorrichtung (100) nach einem der vorhergehenden Ansprüche, **dadurch gekennzeichnet, dass** sie außerdem einen Druckwellenadapter (112) umfasst, der den Ausgang des Erzeugungsmittels (102) an den Eingang (110) des Wellenleitermittels (104) anpasst.

6. Vorrichtung nach einem der Ansprüche 1 bis 5, **dadurch gekennzeichnet, dass** der Anpassungsschlauch (106) eine Form aufweist, die angepasst ist an:
- ein Auge des menschlichen oder tierischen Individuums,
- die Nase des menschlichen oder tierischen Individuums,
- den Mund des menschlichen oder tierischen Individuums, oder
- den Anus des menschlichen oder tierischen Individuums.

7. System (200) zur Abbildung von zumindest einem Bereich und/oder Gewebe und/oder Organ des Körpers eines menschlichen oder tierischen Individuums (202), das System umfassend:
- eine Vorrichtung (100) nach einem der vorhergehenden Ansprüche zum Versetzen des Organs und/oder Gewebes und/oder Bereichs in Schwingung,
- Magnetresonanzabbildungsmittel (204) zum Abbilden der Verschiebungen des Organs und/oder Gewebes und/oder Bereichs, während das Organ und/oder Gewebe und/oder der Bereich in Schwingung versetzt wird.

8. System (300) zur Charakterisierung der mechanischen Eigenschaften von zumindest einem Bereich und/oder Gewebe und/oder Organ des Körpers eines menschlichen oder tierischen Individuums (202), das System umfassend:
- ein System (200) nach Anspruch 7 zum Vorsehen der Verschiebungen innerhalb des Organs und/oder Gewebes und/oder Bereichs,
- zumindest ein computerausführbares Programm zum Analysieren der Verschiebungen zur Charakterisierung der mechanischen Eigenschaften von zumindest einem Teil des Organs und/oder Gewebes und/oder Bereichs.

9. Verfahren (400) zum Einleiten einer mechanischen Welle in zumindest einen Bereich und/oder Gewebe und/oder ein Organ des Körpers eines menschlichen oder tierischen Individuums (202), wobei das Verfahren die folgenden Schritte umfasst:
- Erzeugen (402), durch Erzeugungsmittel (102), einer Druckwelle mit einer gegebenen Frequenz in Luft oder jeglichem anderen Gasgemisch, das zum Beatmen des menschlichen oder tierischen Individuums (202) benutzt werden kann, und
- Leiten (404) der Druckwelle vom Erzeugungsmittel (102) zu einem menschlichen oder tierischen Körper (202) in Luft oder jeglichem anderen Gasgemisch, das zum Beatmen des menschlichen oder tierischen Individuums (202) benutzt werden kann, **dadurch gekennzeichnet, dass** die erzeugte Welle in einem gasförmigen Medium ohne eine mechanische Übertragung mittels fester Medien zum menschlichen oder tierischen Körper übertragen wird.

10. Verfahren (400) zum Einleiten einer mechanischen Welle in zumindest einen Bereich und/oder Gewebe und/oder ein Organ eines menschlichen oder tierischen Körpers (202) nach Anspruch 9, umfassend den Schritt des Anpassens der Länge der Röhre an die Erregungsfrequenz, sodass die Länge der Röhre, neben der Länge des Anpassungsschlauchs, einem ungeraden Mehrfachen einer Viertelwellenlänge der Druckwelle entspricht.

11. Nutzung eines Verfahrens nach Anspruch 9 zum Erregen von Auge, Gehirn, Luftwegen, Herz, Lunge, Prostata oder Gebärmutter eines menschlichen oder tierischen Individuums.

12. Verfahren (400) zur Abbildung eines Organs und/oder von Gewebe und/oder eines Bereichs eines Körpers (202) eines menschlichen oder tierischen Individuums, wobei das Verfahren die folgenden Schritte umfasst:
- Versetzen (402, 404) des Organs und/oder Bereichs in Schwingung gemäß dem Verfahren nach Anspruch 9,
- Magnetresonanzabbilden (406, 408) des Organs und/oder Gewebes und/oder Bereichs, während das Organ und/oder Gewebe und/oder der Bereich in Schwingung versetzt werden.

13. Nutzung eines Verfahrens nach Anspruch 12 zum Abbilden von Auge, Gehirn, Luftwegen, Herz, Lunge, Prostata oder Gebärmutter eines menschlichen oder tierischen Individuums.

14. Verfahren (400) zur Charakterisierung eines Organs und/oder Gewebes und/oder Bereichs eines Körpers (202) eines menschlichen oder tierischen Individuums, wobei das Verfahren die folgenden Schritte umfasst:
- Abbilden (408) von Gewebeverschiebungen des Organs und/oder Bereichs gemäß Anspruch 11, und
- Analysieren (410) der Verschiebungsabbildungen zur Charakterisierung der mechanischen Eigenschaften von zumindest einem Teil des Organs und/oder Gewebes und/oder Bereichs.

15. Nutzung eines Verfahrens nach Anspruch 14 zur Charakterisierung der mechanischen Eigenschaften von Auge, Gehirn, Luftwegen, Herz, Lunge, Prostata oder Gebärmutter eines menschlichen oder tierischen Individuums.

16. Verfahren (1800) zur Charakterisierung eines Organs und/oder Gewebes und/oder Bereichs eines Körpers (202) eines menschlichen oder tierischen Individuums, wobei das Verfahren die folgenden Schritte umfasst:
- Abbilden (1802 - 1808) von Gewebeverschiebungsfeldern des Organs und/oder Bereichs gemäß Anspruch 12, und
- Analysieren (1810) der Verschiebungsfelder zur Charakterisierung der Gewebeanisotropie oder Faserausrichtung von zumindest einem Teil des Organs und/oder Gewebes und/oder Bereichs.

17. Verfahren (1800) nach Anspruch 16, außerdem umfassend einen Schritt (1812) des Analysierens der Verschiebungsfelder und Gewebeanisotropie zur Charakterisierung der anisotropen mechanischen Eigenschaften von zumindest einem Teil des Organs und/oder Gewebes und/oder Bereichs.

## Revendications

1. Appareil (100) pour induire une onde mécanique dans au moins une région du corps et / ou de l'organe et / ou du tissu d'un sujet humain ou animal (202), ledit appareil comprenant:
- un moyen (102) de génération d'une onde de pression d'une fréquence donnée dans un milieu gazeux, et
- un moyen de guide d'ondes (104) sous la forme d'un tube de transmission pour guider, dans l'air ou tout autre mélange gazeux qui peut être utilisé pour ventiler sur le sujet humain ou animal, ladite onde de pression depuis ledit moyen de génération (102) vers un corps humain ou animal (202)
- un tuyau d'adaptation (106), disposé sur l'extrémité (108) du guide d'ondes (104) du côté du corps humain ou animal, pour adapter cette extrémité (108) du guide d'ondes (104) vers une surface ou une cavité dudit corps (202),
**caractérisé en ce que** l'onde générée est transmise au corps humain ou animal dans un milieu gazeux sans transmission mécanique au moyen d'un support solide.

2. Appareil (100) selon la revendication 1, **caractérisé en ce que** le milieu gazeux inclut un gaz marqué pour l'imagerie médicale, comme l'hélium-3 ou l'hexafluorure de soufre pour l'IRM.

3. Appareil (100) selon l'une quelconque des revendications précédentes, **caractérisé en ce que** le moyen pour générer une onde de pression comprend:
- un haut-parleur (118),
- un vibrateur électromécanique, ou
- un élément piézoélectrique.

4. Appareil (100) selon l'une quelconque des revendications précédentes, **caractérisé en ce que** le guide d'ondes sous la forme d'un tube de transmission est un guide d'ondes tubulaire rigide ou flexible (104), dont la longueur et le diamètre sont déterminés en fonction de la fréquence de l'onde de pression.

5. Appareil (100) selon l'une quelconque des revendications précédentes, **caractérisé en ce qu'**il comprend également un adaptateur d'onde de pression (112) adaptant la sortie du moyen de génération (102) à l'entrée (110) du moyen de guide d'ondes (104).

6. Appareil selon l'une quelconque des revendications 1 à 5, **caractérisé en ce que** le tuyau d'adaptation (106) présente une forme adaptée à:
- un œil dudit sujet humain ou animal,
- au nez dudit sujet humain ou animal,
- la bouche dudit humain ou sujet animal, ou
- l'anus dudit sujet humain ou animal.

7. Système (200) pour la cartographie d'au moins une région et / ou un tissu et / ou un organe du corps d'un sujet humain ou animal (202), ledit système comprenant:
- un appareil (100) selon l'une quelconque des revendications précédentes pour mettre en vibration ledit organe et / ou tissu et / ou région,
- un moyen d'imagerie par résonance magnétique (204) pour produire une image des déplacements dudit organe et / ou tissu et / ou région pendant que ledit organe et / ou tissu et / ou région est mis en vibration.

8. Système (300) pour caractériser les propriétés mécaniques d'au moins une région et / ou un tissu et / ou un organe du corps d'un sujet humain ou animal (202), ledit système comprenant:
- un système (200) selon la revendication 7, fournissant les déplacements à l'intérieur dudit organe et / ou tissu et / ou région,
- au moins un programme exécutable par ordinateur pour analyser lesdits déplacements pour caractériser les propriétés mécaniques d'au moins une partie dudit organe et / ou tissu et / ou région.

9. Procédé (400) pour induire une onde mécanique dans au moins une région et / ou un tissu et / ou un organe d'un corps humain ou animal (202), ledit procédé comprenant les étapes suivantes:
- générer (402), par le moyen de génération (102), une onde de pression d'une fréquence donnée dans l'air ou tout autre mélange gazeux qui peut être utilisé pour ventiler le sujet humain ou animal (202), et
- guider (404) ladite onde de pression depuis ledit moyen de génération (102) vers ledit corps humain ou animal (202) dans l'air ou tout autre mélange gazeux qui peut être utilisé pour ventiler le sujet humain ou animal (202),
**caractérisé en ce que** l'onde générée est transmise au corps humain ou animal dans un milieu gazeux sans transmission mécanique au moyen d'un support solide.

10. Procédé (400) pour induire une onde mécanique dans au moins une région et / ou un tissu et / ou un organe d'un corps humain ou animal (202) selon la revendication 9, comprenant l'étape d'adapter la longueur du tube à la fréquence d'excitation, pour que ladite longueur, en plus de la longueur du tuyau d'adaptation, corresponde à un multiple impair d'un quart de longueur d'onde de l'onde de pression.

11. Utilisation d'un procédé selon la revendication 9 pour exciter l'œil, le cerveau, les voies respiratoires, le cœur, le poumon, la prostate ou l'utérus d'un sujet humain ou animal.

12. Procédé (400) pour cartographier un organe et / ou un tissu et / ou une région du corps d'un sujet humain ou animal (202), ledit procédé comprenant les étapes suivantes:
- faire vibrer (402, 404) ledit organe et / ou région selon le procédé selon la revendication 9,
- produire une image par résonance magnétique (406, 408) dudit organe et / ou tissu et / ou région pendant que ledit organe et / ou tissu et / ou région est mis en vibration.

13. Utilisation d'un procédé selon la revendication 12 pour cartographier l'œil, le cerveau, les voies respiratoires, le cœur, le poumon, la prostate, ou l'utérus d'un sujet humain ou animal.

14. Procédé (400) pour caractériser un organe et / ou un tissu et / ou une région du corps d'un sujet humain ou animal (202), ledit procédé comprenant les étapes suivantes:
- cartographier (408) des déplacements tissulaires dudit organe et / ou région selon la revendication 11, et
- analyser (410) lesdites cartes de déplacement pour caractériser les propriétés mécaniques d'au moins une partie dudit organe et / ou tissu et / ou région.

15. Utilisation d'un procédé selon la revendication 14 pour caractériser les propriétés mécaniques de l'œil, du cerveau, des voies respiratoires, du cœur, du poumon, de la prostate ou de l'utérus d'un sujet humain ou animal.

16. Procédé (1800) pour caractériser un organe et / ou un tissu et / ou une région du corps d'un sujet humain ou animal (202), ledit procédé comprenant les étapes suivantes:
- cartographier (1802-1808) des champs de déplacement tissulaire dudit organe et / ou région selon la revendication 12, et
- analyser (1810) lesdits champs de déplacement pour caractériser l'anisotropie tissulaire ou l'orientation des fibres d'au moins une partie dudit organe et / ou tissu et / ou région.

17. Procédé (1800) selon la revendication 16, comprenant également une étape (1812) pour analyser les champs de déplacement et l'anisotropie tissulaire pour caractériser les propriétés mécaniques anisotropes d'au moins une partie de ladite organisation et / ou tissu et / ou région.
